# EUROPEAN PATENT APPLICATION

(11) **EP 0 730 034 A1**
(43) Date of publication of application: **04.09.1996**
(21) Application number: 96102563.2
(22) Date of filing: 21.02.1996
(51) Int. Cl.: C12P 25/00, C07D 475/14

(54) **Purification of riboflavin**

(30) Priority: 03.03.1995 EP 95103025
(71) Applicant: F. HOFFMANN-LA ROCHE AG, 4070 Basel (CH)
(72) Inventor: Kupfer, Ernst, 8006 Zürich (CH)
(74) Representative: Kellenberger, Marcus, Dr.

(57) **Abstract**

A process for the recovery and purification of riboflavin from a post-fermentation broth is characterized by the essential steps of (i) heating said post-fermentation broth at a temperature of about 45°C to about 120°C over a duration of about 10 minutes to about 2 hours for pasteurization, (ii) centrifuging the so-pasteurized broth one or more times to obtain a product consisting largely of riboflavin, (iii) treating said product with aqueous mineral acid at a temperature of about 80°C to about 130°C over a duration of about 30 minutes to about 24 hours and (iv) collecting the riboflavin from the aqueous acid medium of the previous step by filtration and washing it with water, and the optional step of (v) drying the collected washed product of the previous step, to afford riboflavin of substantial purity. A further process for the (further) purification of riboflavin which has been recovered from a post-fermentation broth and optionally purified already to a certain extent by treatment with aqueous mineral acid, filtration and washing with water, e.g. riboflavin recovered after steps (i) and (ii) or after all steps (i) to (iv) and optionally (v), is characterized by the essential steps of (vi) crystallizing the said riboflavin from concentrated hydrochloric acid and water, (vii) collecting the solid riboflavin from the acid medium of the previous step by filtration and washing it with water, and (viii) drying the collected washed product of the previous step, to afford riboflavin of high purity. As a further aspect of this invention the step (i) may be augmented or replaced by the step of adjusting the pH value of the post-fermentation broth to a value of about 5 to 7 by addition of mineral acid.

## Description

The present invention concerns a process of recovery and purification of riboflavin obtained by fermentation.

As is known, riboflavin (vitamin B₂) has valuable health-maintaining, medicinal (e.g. against beri-beri) and growth-promoting properties, and consequently is widely utilized as an additive for foodstuffs and feedstuffs. It is also useful as a yellow colouring agent for foodstuffs. Furthermore, it is known to be producible synthetically and by various fermentation methods, notably by culturing, amongst others, the microorganism Bacillus subtilis, Ashbya gossypii or Eremothecium ashbyii in an appropriate nutrient medium. Depending on the particularly production method, including the isolation/recovery and purification procedure, adopted, the so-obtained riboflavin may satisfy certain quality requirements, e.g. for use in animal feedstuffs, but not others, particularly those for human use. However, in the past the aspired quality for an intended use has not been readily achieved despite intensively investigated post-synthesis or -fermentation isolation/recovery and purification procedures.

It is an object of the present invention to provide a process for the recovery and purification of riboflavin which has been produced by a fermentation method, i.e. is present in the so-called fermentation broth after the fermentation has been effected to a satisfactory extent, preferably to completion (said broth being hereinafter referred to as the "post-fermentation broth"), which process affords a riboflavin product of at least an acceptable quality appropriate to its intended subsequent use.

The present invention provides a process for the recovery and purification of riboflavin from a post-fermentation broth, characterized by the essential steps of
(i) heating said post-fermentation broth at a temperature of about 45°C to about 120°C over a duration of about 10 minutes to about 2 hours for pasteurization,
(ii) centrifuging the so-pasteurized broth one or more times to obtain a product consisting largely of riboflavin,
(iii) treating the product of the previous step with aqueous mineral acid at a temperature of about 80°C to about 130°C over a duration of about 30 minutes to about 24 hours and
(iv) collecting the riboflavin from the aqueous acid medium of the previous step by filtration and washing it with water, and the optional step of
(v) drying the collected washed product of the previous step, to afford riboflavin of substantial purity.

The riboflavin recovered and purified in this way generally has a purity of at least 96%, and is consequently very suitable, after formulation with appropriate additives and processing, such as by spray-drying, for animal feed applications.

Also provided by the present invention is a process for the (further) purification of riboflavin which has been recovered from a post-fermentation broth and optionally purified already to a certain extent by treatment with aqueous mineral acid, filtration and washing with water, which process is characterized by the essential steps of
(vi) crystallising the said riboflavin from concentrated hydrochloric acid and water,
(vii) collecting the solid riboflavin from the acid medium of the previous step by filtration and washing it with water, and
(viii) drying the collected washed product of the previous step, to afford riboflavin of high purity.

The starting riboflavin for (further) purification in this way, i.e. involving the above-indicated steps (vi), (vii) and (viii), is preferably that recovered and purified by the earlier mentioned process involving steps (i), (ii), (iii) and (iv), and optionally also (v). However, it may also be the riboflavin obtained after effecting steps (i) and (ii) only. The product is of high purity, i.e. generally of at least 98%, and is consequently very suitable for human use, particularly in foodstuffs and pharmaceutical applications.

The fermentation producing the riboflavin to be recovered and purified according to the processes of the present invention can itself be effected by any pertinent means. Known fermentation methods involve the culturing of such riboflavin-producing microorganisms as Bacillus subtilis (hereinafter B. subtilis), Ashbya gossypii, Eremothecium ashbyii, Candida flaveri, Clostridium acetobutylicum, Saccharomyces cerevisiae, Brevibacterium ammoniagenes and Torulopsis xylinus in an appropriate nutrient medium, usually under aerobic conditions. The actual fermentation method utilized for producing the riboflavin is immaterial to the present invention. However, as far as the preliminary conditions for the processes of the present invention are concerned, B. subtilis is the preferred microorganism cultured to produce riboflavin by fermentation. Whatever the fermentation method, the techniques for establishing that the fermentation has been effected to a satisfactory extent, preferably to completion, are also well-known, and include HPLC.

The starting point of the processes of the present invention is the aforementioned post-fermentation broth. Said broth is heated according to step (i) at temperature(s) in the range from about 45°C to about 120°C for about 10 minutes to about 2 hours. In general, the higher the temperature up to about 120°C, the less time, as little as about 10 minutes, is required to effect the "pasteurization" in this way. The effect of the heating or pasteurization under these conditions is to inactivate the microorganism remaining from the prior fermentation, to prevent undue broth autolysis and to maintain or create a relatively low viscosity of the broth over a prolonged period of time, as a result of which the subsequent centrifugation(s) [step (ii)] can be effected more readily and efficiently. During the heating, which is preferably effected within the narrower temperature range of about 50°C to about 70°C, the post-fermentation broth is advantageously stirred. Confining the heating duration to less than about 45 minutes is also preferred. Although the pH value of the post-fermentation broth need not be changed before the heating step (i), this may if desired be effected to bring it into the range from about 5 to about 7 by addition of an appropriate amount of mineral acid, preferably concentrated hydrochloric or sulphuric acid. Such acidification serves the same purpose as the heating, and in fact can even replace the heating step (i).

The next process step, (ii), involves one or more centrifugations of the pasteurized post-fermentation broth. In the normal course of events of transferring the broth to the centrifugation equipment, optionally involving an intermediate storage at ambient temperature, the broth will have cooled down considerably, especially to a temperature in the range from about 40°C to about 20°C. However such cooling is not essential, and no special measures need be taken to realize such a cooling. Preferably, however, the broth is within the above-mentioned temperature range immediately prior to the (first) centrifugation. This broth is in the form of a suspension of solid riboflavin and biomass in an aqueous culture medium containing dissolved nutrient material, salts etc. from the preceding fermentation.

The centrifugation itself serves to separate the denser riboflavin from the lighter biomass, whereby between repeated centrifugations the separated denser layer is conveniently suspended in deionized water for the further centrifugation. After each such centrifugation the separated denser layer is richer in riboflavin than that of the previous centrifugation. If more than one centrifugation is performed, the amount of deionised water which is added to the solid (riboflavin) layer separated (or discharged) from the centrifuge for the further centrifugation is suitably about two to six times the weight of said separated or discharged solid layer. As indicated hereinabove, the temperature of the mixture of riboflavin, biomass and aqueous medium which is centrifuged is not critical, and can be in the range from about 15°C to about 80°C, but for practical purposes it is, during the sole or the first of several centrifugations, normally in the range from about 20°C to about 40°C. During subsequent centrifugations the temperature of the centrifuged mixture is preferably ambient temperature. With regard to the relative centrifugal force (RCF) employed in the centrifugation(s), the or the first centrifugation is preferably effected at an RCF in the range of about 2500 x g to about 3000 x g, preferably of about 2600 x g to about 2800 x g. Subsequent centrifugations are preferably effected at an RCF in the region of from about 600 x g to about 750 x g. Suitable centrifugation equipment, especially so-called centrifuge decanters such as the Flottweg Z1L_{d} and the Flottweg Z18-3, is commercially available and well-suited to the purpose at hand, and in such equipment the parameters, e.g. the differential speed between bowl and scroll, the weir diameter and the flux rate of the feed or liquid discharge, can be varied in relation to each other to achieve optimum results. It has been found that 2-4 centrifugations, particularly 3 centrifugations, suffice to afford, after separation, a riboflavin material of sufficient purity to render it in a suitable condition for further purification in the subsequent process steps.

The riboflavin material separated at the completion of the above-described process steps (i) and (ii) has a purity, e.g. after three centrifugations, generally in the region of about 90%.

Step (iii) of the process according to the present invention involves heating the riboflavin separated after the previous centrifugation(s) with aqueous mineral acid under the aforementioned temperature and time conditions, whereby, as in the case of step (i), the actual means of heating is immaterial. Conveniently, however, said heating is effected using superheated steam at a pressure in the range of about 6 to about 10 bar. Furthermore, the higher the heating temperature up to about 130°C, the less time, as little as about 30 minutes, is required. Various preferred heating temperature range and duration combinations have been established, viz. about 90°C to about 130°C over a duration of about 30 minutes to about 6 hours, particularly about 100°C to about 125°C for about 1 to 3 hours; and about 80°C to about 100°C over a duration of about 8 to about 16 hours. In principle any mineral acid can be employed in this process step, e.g. hydrochloric acid, sulphuric acid, nitric acid, phosphoric acid, acetic acid and mixtures of such acids, but preferably hydrochloric acid is used. The acid (e.g. HCl) concentration in the aqueous mineral acid is conveniently from about 0.1 to about 2% by weight, and the concentration of riboflavin in the aqueous mineral acid is conveniently from of about 3 to about 10 percent by weight. The actual technique of bringing the riboflavin in contact with the mineral acid conveniently involves suspending the riboflavin in water, and then adding concentrated acid, or suspending the riboflavin in somewhat less concentrated acid and adding water to the suspension. The water used is preferably deionized. Then the suspension is heated, conveniently with stirring.

The purpose of the above-described process step (iii) is to hydrolyse the biomass present as an undesired impurity and to thereby convert it into a soluble form in the aqueous mineral acid. At the same time any DNA present is also degraded (hydrolysed), and high molecular weight impurities, such as proteins and polysaccharides, are decomposed and also converted into soluble forms. The riboflavin remains in substantially undissolved form on completion of this process step.

In the next process step, (iv), the riboflavin substantially freed of biomass and other impurities through the performance of the previous, acid treatment, step is separated from the aqueous acid medium by filtration and washed with water. The filtration can be performed with the medium at room temperature or at elevated temperatures up to about 125°C or even if possible up to about 130°C. It is convenient to effect the filtration step (iv) directly after completion of the preceding acid treatment step, i.e. without allowing any (significant) cooling between these two steps, and indeed this represents a preferred feature of the present invention. The filtration is also preferably effected in the temperature range from about 70°C to about 90°C, independently of whether or not the preceding acid treatment step was also effected at the same temperature. Depending on the nature of the filter means (which is not material to the present invention) and other factors the filtration may take about three to four hours. After the riboflavin has been so collected, it is washed, optionally repeatedly, with water, preferably deionized water, conveniently either on the filter means or by suspension in water, preferably deionized water, and refiltration. In any event, the washing water itself may be at room temperature or warmer, even up to about 80°C.

The optional drying step (v) is generally performed if the riboflavin is destined for formulation into animal feeds or additives therefor. Such drying is conveniently performed at temperatures in the range from about 70°C to about 90°C, preferably at about 80°C, under reduced pressure, preferably under pressures from about 60 to about 30 mmHg. As indicated hereinabove, the riboflavin at this stage generally has a purity of at least 96%. If, however, the riboflavin is to be purified further, especially where human use is intended, the drying step (v) may be omitted in favour of the three above-defined further process steps (vi), (vii) and (viii).

Process step (vi) involves the crystallization of the still to be further purified riboflavin from concentrated hydrochloric acid and water, and is conveniently effected by dissolving the riboflavin in the minimum, or at least not much more than the minimum, quantity of concentrated hydrochloric acid at the particular temperature selected, and then promoting the precipitation, or more particularly crystallization, of the riboflavin by contacting the solution of riboflavin in concentrated hydrochloric acid with water, preferably deionized water. Said contacting is conveniently effected by adding the solution, e.g. by pouring, into the water. The dissolution of the riboflavin in the hydrochloric acid may be effected at room temperature or above, e.g. at temperatures up to about 80°C, preferably however at room temperature. The hydrochloric acid preferably has a concentration of from about 25% to about 35%. If desired, such agents as absorption charcoal and/or filtration aids, e.g. cellulose or diatomaceous earth, can be added to the solution, which is then filtered before contact with water. The volume ratio of the hydrochloric acid solution of riboflavin to the water conveniently lies in the range from about 1:5 to about 1:15, preferably from about 1:7 to about 1:12. The precipitation/crystallization is suitably allowed to occur at temperatures from about 20°C to about 95°C, preferably at about 80°C.

Thereafter the suspension of riboflavin in aqueous acid medium resulting from the previous process step is filtered and the so-collected riboflavin washed with water, in accordance with process step (vii). This may be carried out analogously to the above-described process step (iv), whereby however temperatures exceeding about 40°C are not recommended. The filtration and washing are preferably effected with the temperature of the media in the range of about room temperature to about 40°C.

Finally, the riboflavin collected in process step (vii) is dried [process step (viii)]. This, too, is conveniently effected under analogous conditions to those of a previous process step, i.e. step (v), described hereinabove. The so purified riboflavin has a high purity, generally of at least 98%, and can be used directly for processing into foodstuffs or pharmaceuticals, as desired.

A further aspect of the present invention resides in a variant of the above-described process comprising the steps (i) to (iv) and optionally (v), whereby the variant entails augmenting or replacing the first step (i) with the step (ia) of adjusting the pH value of the post-fermentation broth to a value in the range from about 5 to about 7 by addition of an appropriate amount of mineral acid, e.g. hydrochloric acid, sulphuric acid, nitric acid or phosphoric acid, preferably (concentrated) hydrochloric or sulphuric acid. During such addition the post-fermentation broth is suitably stirred.

In general, each process step can be effected batchwise, or two or more consecutive steps can be carried out as parts of a continuous procedure. An example of the latter are the continuous acid treatment and filtration steps [(iii) and (iv)] involving no intermediate cooling, as described above.

The invention is illustrated by the following Examples:

### Example 1

Riboflavin was produced by fermentation using B. subtilis in known manner. The post-fermentation broth was then heated at about 60°C for about 30 minutes for pasteurization.

A part (approx. 6,900 l) of the post-fermentation broth was centrifuged by a decanter (Tanabe Z3LL-V). Centrifugation was performed at a relative centrifugal force (RCF) of 2,800 x g, differential speed between bowl and scroll (DS-BS) of 10 rpm and impeller diameter (ID) of 240 mm. The post-fermentation broth was fed to the decanter at the rate of 2,700 l/h. Solid discharge 1 (riboflavin purity: 55.7%) was processed to the next centrifugation step.

Solid discharge 1 (approx. 1,180 kg) was suspended with process water in a 5 kl fermenter suspending vessel to give 3,330 l of suspension. After stirring for 30 minutes the suspension was fed to a decanter at a rate of 1,500 l/h. Centrifugation was performed at a RCF of 2,800 x g, DS-BS of 10 rpm and ID of 240 mm. Solid discharge 2 (riboflavin purity: 76.9%) was processed to the next step.

Solid discharge 2 (approx. 740 kg) was suspended with process water in a 5 kl fermenter suspending vessel to give 3,330 l of suspension. After stirring for 30 minutes, the suspension was fed to the decanter at a rate of 1,200 l/h. Centrifugation was mainly performed at a RCF of 1,630 x g, DS-BS of 8 rpm and ID of 240 mm. Solid discharge 3 weighed 552 kg and had a moisture content of 72.6% and a riboflavin purity of 87.4%.

Approx. 550 kg of riboflavin slurry (solid discharge 3) were suspended in deionized water and hydrochloric acid in a 4 kl glass-lined vessel. The concentration of solid and hydrochloric acid were adjusted to a solid content of 3.9% and a HCl content of 0.6% by weight. The riboflavin suspension was heated to about 120-125°C and maintained in this temperature range for approx. 60 minutes with stirring, and thereafter cooled to 80°C.

The acid-treated suspension of the previous step was filtered using a rubber-lined centrifuge (Mitsubishi Krauss-Maffei, Peeler Centrifuge Type HZ 125). The riboflavin cake was washed with 2,000 l of deionized water at 80°C followed by 200 l of deionized water at 20-30°C. Approx. 190 kg of wet "riboflavin 96%" was obtained with a moisture content of 30.5% and a riboflavin purity of 97%.

338 l of 35% hydrochloric acid and 115 l of deionized water were mixed in a 3 kl mixing vessel, and 180 kg of wet "riboflavin 96%" were charged into the hydrochloric acid solution. The mixture was stirred for one hour. Sample solution was taken from the vessel and dissolution of the riboflavin was confirmed. 1 kg of activated carbon (Takeda Shirasagi-A, 50% wet) was introduced into the mixing vessel and the solution was stirred for one hour. Then 0.6 kg of filter aid (Nippon Paper Industries KC-Floc, cellulose) was added, and the mixture was stirred for 30 minutes. The mixture was then transferred from the mixing vessel into a rubber-lined dissolving vessel for filtration.

Two rubber-lined sparkler filters were prepared for filtration. The first filter contained filter paper Toyo No. 424, the second filter pads Toyo NA10. Then the hydrochloric acid solution of riboflavin in the rubber-lined dissolving vessel was fed into the sparkler filters with pressurized air, and the filtrate was collected in a holding tank. After confirmation of no contamination with the activated carbon in the filtrate, the filtrate in the holding tank was transferred to the dissolving vessel. Then the filtrate was transferred into a filtrate tank. The solution remaining in the filters was transferred with pressurized air to the filtrate tank. The dissolving vessel and the filters was washed with a mixture of 130 l of 35% hydrochloric acid and 60 l of deionized water. The filtered wash solution was also transferred into the filtrate tank. Sample solution was taken from the filtrate tank, and no contamination of activated carbon could be detected.

7,500 l of deionized water were charged into a 17 kl glass-lined crystallization vessel and heated to 78-82°C. The hydrochloric acid solution of riboflavin in the filtrate tank was poured into the crystallizer at a rate of 300-350 l/h, which resulted in the riboflavin crystallizing in the hot water. The mixture was stirred for one hour and then cooled down without stirring. After the temperature of the mixture had cooled to 50°C, stirring was recommenced, and the mixture was further cooled to below 40°C.

1,300 l of deionized water was charged into a 2 kl rubber-lined reslurry tank. The riboflavin slurry in the crystallizer was fed to a string discharge filter. The filtered riboflavin crystals were washed with deionized water and separated into the reslurry tank, and the filtrate was discharged to the waste water treatment plant.

The suspended riboflavin crystals were filtered off by a centrifuge (Mitsubishi Krauss-Maffei, Peeler Centrifuge Type HZ 125) at 950 rpm and washed with 500 l of deionized water. Approx. 140 kg of filter cake were collected in a container and transferred into a 10 kl conical dryer.

The filter cake of riboflavin was charged into the conical dryer and drying carried out at 68-72°C under 60-35 mmHg pressure for 8 hours. Then the dryer was cooled to below 35°C and approx. 90 kg of dry powder could be collected in plastic bags. The product is designated as "riboflavin 98%".

### Analytical results:

The purities of riboflavin 96% and 98% are shown in Table 1. The content of recrystallized riboflavin was 100.2% by photometric assay (E value = 328). As for impurities, 6,7-dimethyl-8-ribityllumazine (DMRL) was removed from riboflavin 98%, while 8-hydroxymethylriboflavin was formed during recrystallization. Lumichrome was detected in both riboflavin 96% and 98%. Compared with synthetic riboflavin 98%, the riboflavin 98% achieved as described above contains less impurities according to chromatographic evidence. The quality of riboflavin 98% met the specifications of EP/BP and USP.

**Table 1**

| | **"Riboflavin 96%"** | **"Riboflavin 98%"** |
|---|---|---|
| Purity: | | |
| Photometric assay | 97.2% | 100.2% |
| HPLC | 97.0% | 99.5% |

| Impurities: | | |
|---|---|---|
| DMRL | 0.06% | not detected |
| 8-Hydroxymethylriboflavin | not detected | 0.14% |
| Lumichrome | 0.10% | 0.11% |

### Example 2

Riboflavin was produced by fermentation using B. subtilis in known manner. The post-fermentation broth was then heated at about 60°C for about 30 minutes for pasteurization, and thereafter pumped to a broth storage tank for further processing.

The broth was then continuously fed to a first decanter at a rate of approx. 3.0 m³/h. The first centrifugation was performed at a relative centrifugal force (RCF) of 2,800 x g. The solid discharged from the decanter was transferred to a sludge tank and reslurried with process water using an in-line mixer. The slurry was then fed to a second decanter at a rate of approx. 1.5 m³/h. The second centrifugation was performed at a RCF of 700 x g. The solid discharged from the second decanter was reslurried with process water and fed to a third decanter at a rate of approx. 1.5 m³/h. The third centrifugation was performed at a RCF of 700 x g. The third step solid was mixed with deionized water and 35% hydrochloric acid and transferred to a storage tank for acid treatment. 35% hydrochloric acid was pumped from a storage tank. The supernatant streams from the decanters were fed to aqueous receiving tanks and discharged to the waste water treatment plant.

Riboflavin slurry containing 3% of solid and 0.7% of HCl was fed to three glass-lined reactors. The slurry was heated to 125°C for 2 hours and cooled to 80°C in the reactors. The slurry from the reactors was then fed to a continuous vacuum filter at a rate of approx. 2.5 m³/h. The filtered cake was washed with hot deionized water and collected in a mixing tank. The filtrate and wash water were discharged to the waste water treatment plant.

The filtered riboflavin was reslurried with deionized water (approx. 65% wetness) in a mixing tank and continuously fed to a spray-type dryer equipped with air heater, exhaust blower and screw feeder. The dried product was collected in a product hopper at a rate of approx. 70 kg/h.. The product "riboflavin 96%" in the product hopper was fed to a packaging system and packed in bulk bags or bag-in-box containers.

### Example 3

After 48 hours fermentation time with B. subtilis in a fermentation to produce riboflavin the 2000 l fermentation vessel was heated with a steam jacket heater at a steam temperature of 120°C. In this way the post-fermentation broth was heated up for 45 minutes to 60°C, maintained within the temperature range 58-62°C for 30 minutes (pasteurization) and allowed to cool down to 25°C over a period of one hour. Thereafter the pasteurized post-fermentation broth had the following characteristics:

Odour: slightly sour; colour: orange-yellow; density: 1.037 g/ml (at 20°C); pH value: 6.93 (at 20°C); and riboflavin titer: 18.45 g/l (HPLC).

Approx. 900 l of the pasteurized broth were centrifuged on a Flottweg Z 1L_{d} decanter. Start-up was immediately after completion of the pasteurization procedure. The broth was fed to the decanter with the help of a pressure of approx. 0.5 bar set on the fermenter vessel. After adjustment of the parameter settings (differential speed between bowl and scroll, weir diameter, pond depth, drying and pool zones, decanter volume, flux of liquid discharge and relative centrifugal force) the decanter was run at a RCF of 2600 x g for approx. 30 minutes before samples of solid and liquid discharge were taken for analysis. The liquid discharge was collected in a 400 l stirred vessel and adjusted to pH ≧ 11 by addition of 28% aqueous sodium hydroxide solution (1 1/20 l of liquid discharge). After holding the solution for 30 minutes at alkaline pH, the liquid was discharged to the waste water treatment plant and the decanter was cleansed by thoroughly washing with industrial water.

Altogether 61.4 kg of solid discharge (product 1) were obtained after this first centrifugation, and this product was stored at 4°C overnight.

The product (1) of the first centrifugation was resuspended in deionized water contained in a 400 l vessel to give 230 l of suspension, which had the following characteristics:

Odour: slightly sour; colour: orange-yellow; density: 1.028 g/ml (at 20°C); pH value: 7.45 (at 20°C); viscosity: 2.28 cSt (at 20°C); and riboflavin concentration: 52.49 g/l (HPLC).

The riboflavin suspension was introduced into a Flottweg Z1L_{d} decanter by pumping with a Watson-Marlow 701 S/R peristaltic pump (silicon tube, inner ⌀: 10 mm; outer ⌀: 15 mm). After adjustment of the parameter settings the decanter was run at a RCF of 650 x g for approx. 15 minutes before samples of solid and liquid discharge were taken for analysis. The liquid discharge was collected and processed analogously to that from the first centrifugation.

Altogether 45.6 kg of solid discharge (product 2) was obtained after this second centrifugation and this product was immediately processed further.

Product 2 was resuspended in deionized water to give 210 l of suspension, which had the following characteristics:

Odour: slightly sour; colour: orange-yellow; density: 1.021 g/ml (at 20°C); pH value: 8.21 (at 20°C); viscosity: 1.37 cSt (at 20°C); and riboflavin concentration: 54.13 g/l (HPLC).

The riboflavin suspension was introduced into a Flottweg Z1L_{d} decanter by pumping with a Watson-Marlow 701 S/R peristaltic pump (specifications as above). After adjustment of the parameter settings the decanter was run at a RCF of 650 x g for approx. 15 minutes before samples of solid and liquid discharge were taken for analysis. The liquid discharge was collected and processed analogously to that from the previous centrifugations.

Altogether 36.4 kg of solid discharge (product 3) were obtained after this third centrifugation.

Results of repeated runs of the above centrifugations using various parameter settings of the centrifuge decanter included the optimum values as presented in the following Table 2:

**Table 2**

| **Riboflavin isolated after:** | **1st centrifugation** | **2nd centrifugation** | **3rd centrifugation** | **Overall** |
|---|---|---|---|---|
| Yield of riboflavin | 96.8% | 97.6% | 99.1% | 93.5% |
| Purity of riboflavin | 59.7% | 86.5% | 91.3% | 91.3% |
| Throughput | 132 l/h | 114 l/h | 114 l/h | - |

The so-designated "90% riboflavin" obtained after recovery from the post-fermentation broth and purification from the three centrifugations as described above can then be submitted to acid treatment (iii), filtration and washing (iv), crystallization (vi), filtration and washing (vii) and drying (viii) steps analogous to those described in Examples 1 (from the fifth paragraph) and 2 (from the second paragraph) to afford "riboflavin 96%" and/or "riboflavin 98%", as appropriate.

### Example 4

Two control experiments were performed on a laboratory scale with a post-fermentation broth. In the first experiment (A) the post-fermentation broth was acidified to pH 5 with 25% hydrochloric acid, but not heated, prior to riboflavin recovery, and in the second (B) the post-fermentation broth was heated at about 60°C for about 30 minutes (pasteurized) prior to the recovery.

In each case 1 l of post-fermentation broth was centrifuged at a RCF of 1100 x g for 5 minutes (2000 rpm on a Mistral 6000 centrifuge with swing-out rotor 258-963). The supernatant was discharged and the solid denser material (pellet) resuspended in deionized water with stirring for 15 minutes. The so-obtained riboflavin was then centrifuged at a RCF of 630 x g for 5 minutes (1500 rpm on the same centrifuge) and the new supernatant discharged and the pellet resuspended in water. After a final (third) centrifugation in this manner the obtained pellet was dried at 80°C for 20 hours in vacuo and then crushed and again dried for a further 4 hours under these conditions.

At each stage the yield and purity of the pertinent products were determined: 2 ml of riboflavin suspension were dried at 110°C in vacuo for 30 minutes to determine the weight of the dried residue, and 10 ml of riboflavin suspension were taken for analysis of the riboflavin concentration (purity).

The results of these experiments are presented in the following Table 3:

**Table 3**

| | | **Experiment** | |
|---|---|---|---|
| | | **A** | **B** |
| 1st Centrifugation: riboflavin | yield | 95% | 96% |
| | purity | 50% | 54% |
| 2nd Centrifugation: riboflavin | yield | 99% | 100% |
| | purity | 78% | 84% |
| 3nd Centrifugation: riboflavin | yield | 100% | 99% |
| | purity | 89% | 94% |
| Overall Recovery: riboflavin | yield | 95% | 95% |
| | purity | 89% | 94% |

The so-designated "90% riboflavin" obtained after recovery from the post-fermentation broth and purification from the three centrifugations as described above can then be submitted to acid treatment (iii), filtration and washing (iv), crystallization (vi), filtration and washing (vii) and drying (viii) steps analogous to those described in Examples 1 (from the fifth paragraph) and 2 (from the second paragraph) to afford "riboflavin 96%" and/or "riboflavin 98%", as appropriate.

### Example 5

133 g of "riboflavin 90%" (30 g riboflavin, 3 g biomass, 100 g water), 120 ml of 1M hydrochloric acid and 440 ml of deionized water were mixed to a suspension of 5% solids. 50 µl of octyl alcohol were added as an antifoam agent. The slurry was heated at 100°C for 30 minutes with stirring at 300 rpm. However, the stirring was decreased to 75 rpm when the viscosity of the slurry had increased due to changes in the shape of the riboflavin crystals during the heating up to 100°C. The slurry was then transferred to a steam vessel and was heated at 120-125°C for 60 minutes. After cooling down the riboflavin slurry to 80°C it was filtered on a filter paper (Machery and Nagel 713, ⌀ 90 mm) under vacuum. The filter cake was washed with 300 ml of deionized water at 80°C. Riboflavin was reslurried with 300 ml of deionized water. The slurry was heated at 80°C for 15 minutes with stirring. Then the riboflavin slurry was filtered at 80°C on a filter paper under vacuum. The filter cake was washed with 300 ml of deionized water of 80°C. The riboflavin obtained was dried at 80°C in vacuo and was crushed. Such riboflavin product, designated as "riboflavin 96%", generally has a purity of at least 96% and is of high quality.

### Example 6

30 g of "riboflavin 90%" was dissolved in 105 ml of 25% hydrochloric acid (3.5 ml/g riboflavin) with stirring for 10 minutes at room temperature. 0.9 g of Norit SX2 activated charcoal was added. The solution was heated to 80°C for approx. 8 minutes and was then held for 20 minutes at this temperature. The hot solution was filtered under vacuum on a twofold layer of filter paper (Machery and Nagel 713, ⌀ 40 mm) toppered with 1.0 g of Clarcel. The filtration time was 10-70 minutes, depending on the quality of the "riboflavin 90%" used. The filtrate (approx. 117 ml) was introduced at a rate of 2 ml/min. into 840 ml (8 times the volume of acid used) of deionized water at 80°C with stirring (300 rpm). Seeding crystals of riboflavin were added after 5 and 10 minutes. When the precipitation of riboflavin was complete, the crystal slurry was stirred another 30 minutes at 80°C. The slurry was then cooled to approx. 25°C within 60 minutes. The riboflavin slurry was filtered on a filter clother (DACRON DA-50) under vacuum. The filter cake was washed with 300 ml of deionized water and then reslurried with 300 ml of deionized water and stirred for 15 minutes at 23°C. The riboflavin slurry was filtered on a filter cloth under vacuum and the filter cake washed with 300 ml of deionized water, followed by 300 ml of methanol (FLUKA puriss.). The riboflavin obtained was dried at 80°C in a vacuum oven to constant weight and was crushed. Such riboflavin product, designated as "riboflavin 98%", generally has a purity of at least 98% and is of particularly high quality.

### Example 7

30 g of "riboflavin 90%" was dissolved in 105 ml of 25% hydrochloric acid (3.5 ml/g riboflavin) with stirring for 10 minutes at room temperature. The solution was heated to 80°C for approx. 8 minutes and was then held for 20 minutes at this temperature. The hot solution was filtered under vacuum on a twofold layer of filter paper (Machery and Nagel 713, ⌀ 40 mm). The filtration time was 1-6 minutes, depending on the quality of the "riboflavin 90%" starting material. The filtrate (approx. 121 ml) was introduced at a rate of 2 ml/min. into 840 ml (8 times the volume of hydrochloric acid used) of deionized water at 80°C with stirring (300 rpm). Seeding crystals of riboflavin were added after 5 and 10 minutes. When the precipitation of riboflavin was complete, the crystal slurry was stirred another 30 minutes at 80°C. The slurry was then cooled to approx. 25°C within 60 minutes. The slurry was filtered on a filter cloth (DACRON DA-50) under vacuum. The filter cake was washed with 300 ml of deionized water and reslurried with 300 ml of deionized water and stirred for 15 minutes at 23°C. The riboflavin slurry was filtered on a filter cloth under vacuum and the filter cake washed with 300 ml of deionized water, followed by 300 ml of methanol (FLUKA puriss.). The riboflavin obtained was dried at 80°C in a vacuum oven to constant weight and was crushed. Such riboflavin, designated as "riboflavin 98%", generally has a purity of at least 98% and is of particularly high quality.

### Example 8

90.4 g of "riboflavin 90%" (30.74 g riboflavin, 34% impurities and 56.3 g water) were suspended in 120 ml of 1M hydrochloric acid and 460 ml of deionized water and to the suspension were then added 50 µl of the antifoam agent octyl alcohol. The whole was heated at 100°C for 30 minutes with stirring and subsequently at 120-125°C for 60 minutes in a steam vessel.

The suspension was cooled down to 80°C and the crystals collected by filtration through Machery and Nagel (MN) 713 filter paper (⌀ 90 mm) in a filter flask. After the so-collected riboflavin crystals had been washed with 300 ml of deionized water at 80°C they were resuspended in 300 ml of deionized water and the suspension was heated at 80°C for 15 minutes with stirring. Then the crystals were filtered off as before, washed with 300 ml of deionized water at 80°C, dried at 80°C in vacuo for 20 hours and, after being crushed, dried for a further 2 hours in vacuo.

In this way there were obtained 29.07 g of "riboflavin 96%" of seemingly 100% purity in a yield of approx. 95%.

### Example 9

30.0 g of dried "riboflavin 90%" (92.8% purity) were dissolved in 105 ml of 25% (w/v) hydrochloric acid with stirring for 10 minutes, after which 0.9 g of Norit SX2 activated charcoal was added. The mixture was stirred at 80°C for 20 minutes. Then the mixture was filtered through a double layer of MN 713 filter paper (⌀ 40 mm) covered with 1.0 g of Clarcel in a filtering flask.

The filtrate was added at a rate of 2 ml/min. to 840 ml of deionized water at 80°C with stirring and addition of riboflavin crystals for seeding. Precipitation of riboflavin occurred, and the resulting suspension was stirred for a further 30 minutes and then cooled down to 25°C within 60 minutes.

Using a Dacron DA-50 filter doth (⌀ 90 mm) the crystals of riboflavin were filtered off from the suspension in a filtering flask and washed with 300 ml of deionized water at 20°C. The so-collected riboflavin was reslurried in 300 ml of deionized water at 20°C, and the crystals again filtered off and washed as before and additionally with 300 ml of methanol.

After drying the so-collected and -washed riboflavin at 80°C in vacuo to constant weight there were obtained 25.1 g of "riboflavin 98%" of seemingly almost 100% purity in a yield of approx. 93%.

### Example 10

"Riboflavin 90%" is isolated after a 3-step centrifugation and mixed with deionized water and hydrochloric acid in the production plant. Various samples of the so-obtained riboflavin suspended in dilute acid solution are stored at room temperature in a storage tank. Each sample solution was taken from the storage tank and used for an experiment concerning the acid treatment and filtration steps of the invention [steps (iii) and (iv)]. In each case the slurry of riboflavin in dilute hydrochloric acid was heated at 90°C or 120°C under atmospheric pressure in a separable flask for a definite time with stirring, thereafter filtered hot (without cooling), the collected solid washed with hot water, dried at 80°C under vacuum for about 12 hours and the purity of the so-obtained "riboflavin 96%" determined. The pertinent data and results from this batch treatment procedure for steps (iii) and (iv) of the inventive process are presented in the following Table 4:

**Table 4**

| Experiment No. | Determined purity of "riboflavin 90%" | Conditions of acid treatment | | | | Determined purity of "riboflavin 96%" |
|---|---|---|---|---|---|---|
| | | Solids concn. (% by wt.) | Concn. of dil. HCl (% by wt.) | Temp. (°C) | Time (hours) | |
| A | 87.4 | 6.35 | 1.1 | 90 | 8 | 96.2 |
| B | 87.4 | 6.35 | 1.1 | 90 | 10 | 96.2 |
| C | 87.4 | 6.35 | 1.1 | 120 | 1 | 97.4 |
| D | 89.8 | 6.19 | 0.9 | 90 | 12 | 96.0 |
| E | 89.8 | 6.19 | 0.9 | 90 | 14 | 96.4 |
| F | 89.8 | 6.19 | 0.9 | 90 | 16 | 96.8 |
| G | 92.7 | 6.06 | 0.8 | 90 | 10 | 96.3 |
| H | 92.7 | 6.06 | 0.8 | 90 | 12 | 96.5 |
| I | 92.7 | 6.06 | 0.8 | 90 | 14 | 96.8 |

### Example 11

In a further set of experiments, performed according to a continuous procedure of acid treatment and filtration [steps (iii) and (iv) of the inventive process], each sample of "riboflavin 90%" slurry in dilute hydrochloric acid of concentration 0.8% by weight was fed into a heating flask at the rate of 72 ml/hour, heated therein at 90°C, transfered continuously to a second heating flask, also at the rate of 72 ml/hour, and heated in this flask for a further period at 90°C. The heated contents of the second flask were continuously transferred at the rate of 72 ml/hour without cooling to a filter unit and filtered hot. Finally, the collected solid was washed with hot water, dried under vacuum at 80°C for about 12 hours and the purity of the so-obtained "riboflavin 96%" determined. The pertinent data and results from this continuous acid treatment and filtration procedure are presented in the following Table 5:

**Table 5**

| Experiment No. | Determined purity of "riboflavin 90%" | Conditions of acid treatment | | Determined purity of "riboflavin 96%" |
|---|---|---|---|---|
| | | Solids concn. (% by wt.) | Total heating time in two flasks (hours) | |
| A | 92.7 | 5.79 | 14 | 97.0 |
| B | 90.3 | 6.19 | 14 | 96.3 |
| C | 91.3 | 5.66 | 12 | 96.7 |
| D | 91.3 | 5.66 | 13 | 96.7 |
| E | 91.3 | 5.66 | 14 | 97.3 |

## Claims

1. A process for the recovery and purification of riboflavin from a post-fermentation broth, characterized by the essential steps of
(i) heating said post-fermentation broth at a temperature of about 45°C to about 120°C over a duration of about 10 minutes to about 2 hours for pasteurization,
(ii) centrifuging the so-pasteurized broth one or more times to obtain a product consisting largely of riboflavin,
(iii) treating the product of the previous step with aqueous mineral acid at a temperature of about 80°C to about 130°C over a duration of about 30 minutes to about 24 hours and
(iv) collecting the riboflavin from the aqueous acid medium of the previous step by filtration and washing it with water,
and the optional step of
(v) drying the collected washed product of the previous step, to afford riboflavin of substantial purity.

2. A process for the (further) purification of riboflavin which has been recovered from a post-fermentation broth and optionally purified already to a certain extent by treatment with aqueous mineral acid, filtration and washing with water, which process is characterized by the essential steps of
(vi) crystallizing the said riboflavin from concentrated hydrochloric acid and water,
(vii) collecting the solid riboflavin from the acid medium of the previous step by filtration and washing it with water, and
(viii) drying the collected washed product of the previous step, to afford riboflavin of high purity.

3. A process according to claim 2, wherein the riboflavin to be so purified is that recovered from a post-fermentation broth and purified by the process of claim 1.

4. A process according to claim 2, wherein the riboflavin to be so purified is that recovered from a post-fermentation broth by the process steps (i) and (ii) of claim 1.

5. A process according to claim 1, wherein the step (i) is augmented or replaced with the step of adjusting the pH value of the post-fermentation broth to a value in the range from about 5 to about 7 by addition of an appropriate amount of mineral acid.

6. A process according to any preceding claim, wherein the riboflavin to be recovered and/or purified has been produced by fermentation by culturing the microorganism Bacillus subtilis.

7. A process according to any one of claims 1 and 3 to 6, wherein the step (i) is effected by heating the post-fermentation broth at a temperature in the range of about 50°C to about 70°C for less than about 45 minutes.

8. A process according to any one of claims 1 and 3 to 7, wherein the step (ii) consists of three centrifugations.

9. A process according to any one of claims 1, 3 and 5 to 8, wherein the step (iii) is effected by treating the product of step (ii) with aqueous mineral acid at a temperature in the range of about 90°C to about 130°C over a duration of about 30 minutes to about 6 hours, particularly in the temperature range of about 100°C to about 125°C for about 1 to 3 hours.

10. A process according to any one of claims 1, 3 and 5 to 8, wherein the step (iii) is effected by treating the product of step (ii) with aqueous mineral acid at a temperature in the range of about 80°C to about 100°C over a duration of about 8 to about 16 hours.

11. A process according to any one of claims 1, 3 and 5 to 10, wherein the step (iv) is effected directly after completion of the preceding step (iii) without allowing any cooling between these two steps.
